# FASCICULE DE BREVET EUROPEEN

(11) **EP 3 524 163 B1**
(45) Date de publication et mention de la délivrance du brevet: **26.02.2025**
(21) Numéro de dépôt: 19154028.5
(22) Date de dépôt: 28.01.2019
(51) Int. Cl.: A61B 8/00, A61N 7/00, A61B 17/225, A61N 7/02

(54) **SOURCE DE PRODUCTION D'ONDES ULTRASONORES AVEC FILETAGE EXTERIEUR**
QUELLE ZUR ERZEUGUNG VON ULTRASCHALLWELLEN MIT AUSSENGEWINDE
SOURCE FOR PRODUCING ULTRASONIC WAVES WITH EXTERNAL THREAD

(30) Priorité: 12.02.2018 FR 1851140
(43) Date de publication de la demande: 14.08.2019
(73) Titulaire: EDAP TMS France, 69120 Vaulx-en-Velin (FR)
(72) Inventeur: VINCENOT, Jérémy, 69100 VILLEURBANNE (FR); NALLET, Olivier, 69003 LYON (FR); NURY, David, 38300 BOURGOIN JALLIEU (FR)
(74) Mandataire: Cabinet Beau de Loménie

(56) Documents cités:
- EP-A1- 2 327 450
- US-A1- 2004 097 840
- US-A1- 2015 182 200

## Description

La présente invention concerne le domaine technique des appareils ou des dispositifs comportant une source de production d'ondes ultrasonores et utilisés dans le domaine thérapeutique et pouvant éventuellement être associés avec une sonde ultrasonore d'imagerie échographique de l'anatomie humaine.

L'objet de l'invention trouve une application particulièrement avantageuse dans le domaine des appareils de thérapie comportant une source de production d'ondes ultrasonores focalisées à haute intensité (HIFU).

L'objet de l'invention trouve une autre application particulièrement avantageuse dans le domaine des appareils de destruction de concrétions intracorporelles solides pour des traitements de lithotritie comportant une source de production d'ondes ultrasonores de pression telles que des ondes de choc acoustiques permettant de détruire des concrétions ou des lithiases rénales, biliaires ou salivaires.

D'une manière générale, une source de production d'ondes ultrasonores comporte un corps de montage pour un transducteur acoustique qui convertit une énergie électrique en une énergie mécanique. Ce transducteur ultrasonore présente vers l'avant, une face d'émission délimitant avec une membrane souple, une chambre de confinement pour un fluide de couplage acoustique et/ou de refroidissement. Classiquement, cette membrane souple est montée sur un support annulaire du corps à l'aide d'un système de fixation à caractère étanche et démontable pour pouvoir être remplacée après chaque utilisation.

Typiquement, la membrane souple est montée sur le support annulaire à l'aide d'un anneau élastique dont la force de rappel est adaptée pour exercer un effort suffisant afin d'assurer le montage étanche de la membrane sur le support tout en permettant la mise en place de cet anneau avec un effort manuel permettant une mise en place aisée. En pratique, l'opération de montage de cette membrane souple s'avère relativement difficile à mener à bien pour assurer une parfaite étanchéité de la chambre de confinement sur tout le pourtour du support annulaire. De plus, cette opération de montage conduit parfois à une destruction du transducteur ultrasonore par un appui malencontreux réalisé par l'opérateur, compte tenu de la difficulté à mettre en place l'anneau élastique.

La demande de brevet EP 2 327 450 décrit une sonde de production ultrasonore comportant une membrane flexible amovible destinée à venir recouvrir une tête HIFU dans laquelle est monté un transducteur HIFU. Cette sonde est pourvue de moyens de connexion pour réaliser une fixation détachable entre la membrane et la tête HIFU. Ces moyens de connexion comportent une rainure circonférentielle réalisée sur le côté intérieur de la membrane et engageable avec une nervure circonférentielle formée sur le côté extérieur de la tête HIFU. De façon complémentaire, cette membrane comporte sur sa face interne, trois nervures périphériques destinées à être pressées contre la tête HIFU pour assurer une étanchéité et une circulation pour le liquide de refroidissement confiné entre la membrane et la tête HIFU.

Il est à noter que l'opération de montage de la membrane flexible nécessite une déformation élastique de la membrane pour assurer l'engagement de la rainure dans la nervure de la tête. Même si cette membrane est équipée d'une languette de traction, sa mise en place nécessite un effort manuel non négligeable, difficile à contrôler pour assurer une parfaite étanchéité sur tout le pourtour de la tête HIFU.

Dans le domaine des sondes échographiques, le document US 2015/0182200 décrit une sonde échographique comportant une tête de sonde reliée par un faisceau à un connecteur comprenant un système de refroidissement assurant la circulation d'un fluide de refroidissement entre la tête de sonde et le connecteur. Ce système de refroidissement comporte un amortisseur des pulsations de la pression du fluide créées par la pompe et nuisant à la qualité des images ultrasonores. Cet amortisseur des vibrations comporte une membrane souple montée pour délimiter d'un côté, une chambre de réception du fluide de refroidissement et de l'autre côté, une chambre d'air fermé. L'étanchéité entre les deux chambres est assurée par la compression de la membrane prise en sandwich entre le boîtier et une bague vissée sur l'intérieur du boîtier. Les pulsations du fluide de refroidissement sont absorbées par la déformation mécanique de la membrane souple.

Ce document est muet sur la description de la tête de la sonde et sur un éventuel dispositif de montage pour une membrane amovible définissant une chambre de confinement pour le fluide de refroidissement en relation du transducteur ultrasonore.

L'analyse de l'art antérieur conduit à constater qu'il apparaît le besoin de pouvoir disposer d'une sonde ultrasonore adaptée pour pouvoir être équipée d'une membrane souple, sans nécessiter le recours à des outils ou à la réalisation de manipulations difficiles à mener à bien. De façon complémentaire, le transducteur ultrasonore qui se trouve placé à proximité de l'ouverture du boîtier recevant cette membrane, est susceptible d'être détériorée au cours de la mise en place de cette membrane, mais également en dehors de l'utilisation de la sonde ultrasonore. Il apparaît ainsi le besoin de pouvoir protéger le transducteur ultrasonore de la sonde aussi bien pendant son utilisation qu'en dehors de ses périodes d'utilisation, par un système efficace et simple à mettre en place.

Il est à noter que la sonde ultrasonore subit des opérations de calibration nécessitant la mise en œuvre d'un système d'alignement du faisceau ultrasonore. Outre les problèmes de mise en place d'un tel système d'alignement avec le faisceau ultrasonore, cette opération de calibration doit être menée à bien sans risque de détérioration du transducteur ultrasonore.

La présente invention vise à remédier aux inconvénients de l'état de la technique en proposant une nouvelle source de production d'ondes ultrasonores conçue pour optimiser les diverses interventions susceptibles d'intervenir au cours de sa mise en œuvre.

Un objectif de la présente invention est de proposer une nouvelle source de production d'ondes ultrasonores conçue pour faciliter, sans risques de détérioration du transmetteur ultrasonore, les opérations :
- de mise en place de la membrane souple de confinement du fluide de couplage acoustique et/ ou de refroidissement ;
- de protection du transducteur ultrasonore lorsque la sonde n'est pas utilisée ;
- de calibrage du transducteur ultrasonore.

Pour atteindre de tels objectifs, l'invention concerne une source de production d'ondes ultrasonores selon la revendication 1.

Selon un exemple la bague de fixation est pourvue d'une rondelle montée libre en rotation relativement par rapport à la bague de fixation afin de ne pas être entraîner en rotation lors de l'opération de vissage de la bague de fixation, cette rondelle exerçant un effort de pression sur la membrane souple en position vissée de la bague de fixation sur le corps. Un tel dispositif permet d'obtenir un montage de la membrane sans pli sur le boîtier.

De plus, la source de production d'ondes ultrasonores peut comporter également en combinaison l'une et/ou l'autre des caractéristiques additionnelles suivantes :
- la rondelle est montée dans la bague de fixation avec un débattement latéral limité pour exercer un effort de pression sur la membrane souple en position vissée de la bague de fixation sur le corps ;
- la rondelle est maintenue dans la bague de fixation à l'intérieur d'un logement annulaire de section en forme de C ;
- un joint d'étanchéité en contact avec la membrane souple ;
- la rondelle est équipée du joint d'étanchéité ;
- la rondelle est réalisée en polytétrafluoroéthylène ;
- le filet ou le filet complémentaire peut être réalisé sous la forme d'un élément continu ou sous la forme d'éléments discontinus tels que des ergots.

Selon un autre exemple, pour lequel le transducteur ultrasonore est à protéger, le corps comporte un filet coopérant avec un filet complémentaire d'un couvercle de protection.

Selon un autre exemple pour lequel une phase de calibration du transducteur ultrasonore doit intervenir, le corps comporte un filet coopérant avec un filet complémentaire d'un système d'alignement utilisable notamment lors de l'étape de calibration.

La présente invention propose également un appareil de traitement par ondes ultrasonores comportant une source de production d'ondes ultrasonores selon l'une des revendications 1 à 10.

Diverses autres caractéristiques ressortent de la description faite ci-dessous en référence aux dessins annexés qui montrent, à titre d'exemples non limitatifs, des formes de réalisation de l'objet de l'invention.

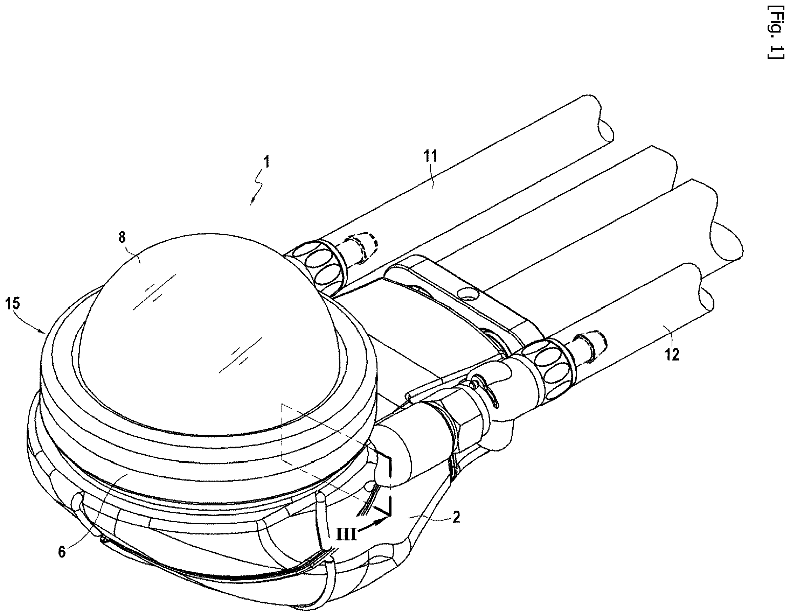

La **Figure 1** est une vue en perspective d'un exemple de réalisation d'une source de production d'ondes ultrasonores mettant en œuvre l'objet de l'invention.
La **Figure 2** est une vue en perspective éclatée d'un exemple de réalisation d'une source de production d'ondes ultrasonores conforme à l'invention.
La **Figure 3** est une vue en coupe élévation prise selon la section III de la source de production d'ondes ultrasonores illustrée à la **Fig. 1****.**
**La** **Figure 4** est une vue en perspective montrant un autre exemple de réalisation d'une source de production d'ondes ultrasonores conforme à l'invention.
La **Figure 5** est une vue en perspective éclatée montrant un exemple de réalisation d'une source de production d'ondes ultrasonores conforme à l'invention destinée à être équipée d'un couvercle de protection.
La **Figure 6** est une vue en perspective éclatée montrant un exemple de réalisation d'une source de production d'ondes ultrasonores conforme à l'invention destinée à être équipée d'une bague munie d'un système d'alignement.

Tel que cela ressort des dessins, l'objet de l'invention concerne une source **1** de production d'ondes ultrasonores pour le traitement thermique de tissus biologiques. Cette source **1** qui est utilisée plus particulièrement pour le traitement thérapeutique, fait partie d'un appareil de thérapie au sens général non représenté mais connu en soi et adapté pour réaliser le traitement de tissus d'un être vivant par l'intermédiaire d'ondes ultrasonores. Selon une application avantageuse, cette source de thérapie produit des ultrasons focalisés de haute intensité (HIFU). Selon une autre application, cette source produit des ondes ultrasonores de pression telles que des ondes de choc acoustiques. Bien entendu, cette source de production d'ondes ultrasonores de thérapie peut être associée ou non, à une sonde d'imagerie échographique de l'anatomie humaine.

La source de production d'ondes ultrasonores **1** comporte notamment un corps **2** de support pour un transducteur **3** comportant un ou plusieurs émetteurs ultrasonores tels que par exemple des éléments piézoélectriques. Les émetteurs ultrasonores du transducteur **3** sont reliés via, un étage amplificateur, à un circuit de commande délivrant des signaux pour activer les émetteurs ultrasonores. Le circuit de commande n'est pas décrit plus précisément car sa réalisation fait partie des connaissances techniques de l'homme du métier. Ce circuit de commande comporte ainsi classiquement un générateur de signal piloté qui est relié aux émetteurs ultrasonores par l'intermédiaire de l'étage amplificateur.

Le transducteur **3** présente à l'avant, une face d'émission **4** des ondes ultrasonores, tournée vers une ouverture **5** aménagée dans le corps **2.** Typiquement, le corps **2** ou boîtier est aménagé pour présenter une paroi tubulaire **6** formant une collerette ou un col. Cette paroi tubulaire **6** du corps est délimitée entre une face externe **6e** et une face interne **6i** et se trouve pourvue à son extrémité d'un rebord annulaire **6a** délimitant intérieurement l'ouverture **5.**

De manière générale, la face d'émission **4** possède un axe de symétrie **X** correspondant à l'axe de propagation des ondes acoustiques passant sensiblement par le centre de l'ouverture **5.** Selon une variante avantageuse de réalisation, la face d'émission **4** présente une géométrie focalisante c'est-à-dire que les ondes ultrasonores produites sont focalisées dans une zone focale soit en raison du mode de commande des émetteurs ultrasonores soit par la forme géométrique de la face d'émission. Typiquement, la face d'émission **4** a une forme concave telle une forme hémisphérique, elliptique ou torique. Bien entendu, la face d'émission **4** peut présenter une forme différente d'une forme concave comme une forme plane par exemple.

Le transducteur ultrasonore **3** se trouve ainsi directement positionné en regard de l'ouverture **5.** Typiquement, le transducteur ultrasonore **3** est situé au niveau ou légèrement en retrait de l'ouverture **5.**

En fonction de la mise en œuvre de la source de production **1** conforme à l'invention, cette source de production **1** est destinée à recevoir divers équipements ou accessoires adaptés chacun à une fonction spécifique.

Lorsque la source **1** est utilisée pour produire des ondes ultrasonores, la source de production d'ondes ultrasonores **1** comporte une membrane souple **8** placée devant la face d'émission **4** et réalisée en un matériau transparent aux ondes ultrasonores. Cette membrane souple **8** délimite avec la face d'émission **4,** une chambre de confinement **10** pour un fluide de refroidissement apte à remplir également une fonction de couplage acoustique avec le milieu insonifié. Typiquement, le fluide de refroidissement est un liquide à base d'eau préalablement dégazée pour améliorer la propagation des ondes ou d'huile sélectionnée parmi celles présentant des caractéristiques acoustiques de faible absorption des ondes ultrasonores. On peut également utiliser le liquide décrit dans le brevet EP 1 038 551.

La chambre de confinement **10** comporte au moins une entrée pour le fluide de refroidissement qui est amené par au moins une canalisation d'alimentation **11.** La chambre de confinement **10** comporte également au moins une sortie communicant avec une conduite de sortie **12** permettant ainsi une circulation du fluide à l'intérieur de cette chambre de confinement.

La membrane souple **8** est réalisée en tous matériaux appropriés tels que du silicone ou du latex ou du polyuréthane ou tous matériaux présentant une faible absorption acoustique afin de réduire les pertes d'énergie acoustiques liées à l'échauffement de la membrane.

La membrane souple **8** est montée sur le corps **2** à l'aide d'un système de fixation **14** à caractère étanche et à caractère amovible ou démontable. Le système de fixation **14** permet de pouvoir changer à volonté, rapidement et aisément la membrane souple **8** tout en retrouvant l'étanchéité de la chambre de confinement **10** après le changement de la membrane.

Conformément à l'invention, le système de fixation **14** comporte une bague de fixation **15** destinée à être assemblée au corps **2** de manière démontable. Cette bague de fixation **15** et le corps **2** sont pourvus d'un système d'assemblage par filetage **16, 17** pour assurer, par le vissage de la bague de fixation **15** sur le corps **2,** le maintien de la membrane souple **8** en appui étanche sur le corps **2.**

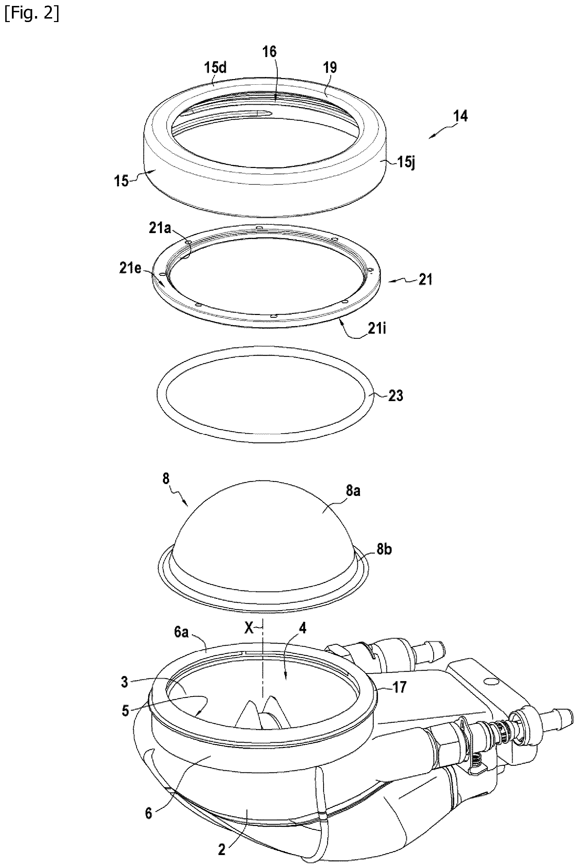

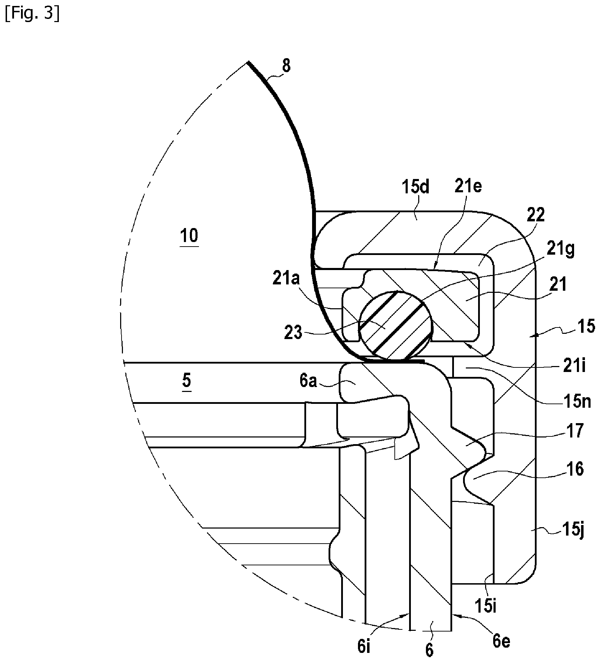

Tel que cela ressort plus précisément des **Fig. 2** et **3****,** la bague de fixation **15** se présente sous la forme d'un corps annulaire comportant une paroi annulaire de dessus **15d** délimitant en son centre, un passage central **19** pour une partie de la membrane souple **8** et en particulier, une partie centrale **8a** de cette membrane souple. La membrane souple **8** et la bague de fixation **15** sont deux pièces indépendantes l'une de l'autre. Typiquement, ce passage central **19** présente une section correspondant sensiblement à la section de l'ouverture **5** de sorte qu'en position montée de la bague de fixation **15** sur le corps **2,** l'ouverture **5** et le passage central **19** coïncident.

Typiquement, le passage central **19** présente un contour circulaire. Bien entendu, la membrane souple **8** possède une forme adaptée ou déformable pour traverser par sa partie centrale **8a,** le passage central **19** et former la chambre de confinement **10.** La partie centrale **8a** de la membrane souple se trouve bordée par une partie périphérique **8b** destinée à coopérer avec le corps **2** pour assurer l'étanchéité à la chambre de confinement **10.** Plus précisément, la partie périphérique **8b** de la membrane souple **8** est adaptée pour venir en appui sur le rebord annulaire **6a** de la paroi annulaire **6.**

La paroi annulaire de dessus **15d** se prolonge à l'équerre par une jupe tubulaire **15j** présentant une face interne **15i** et destinée à s'étendre en relation de la paroi tubulaire **6** du corps **2,** en position montée de la bague de fixation **15** sur le corps **2.**

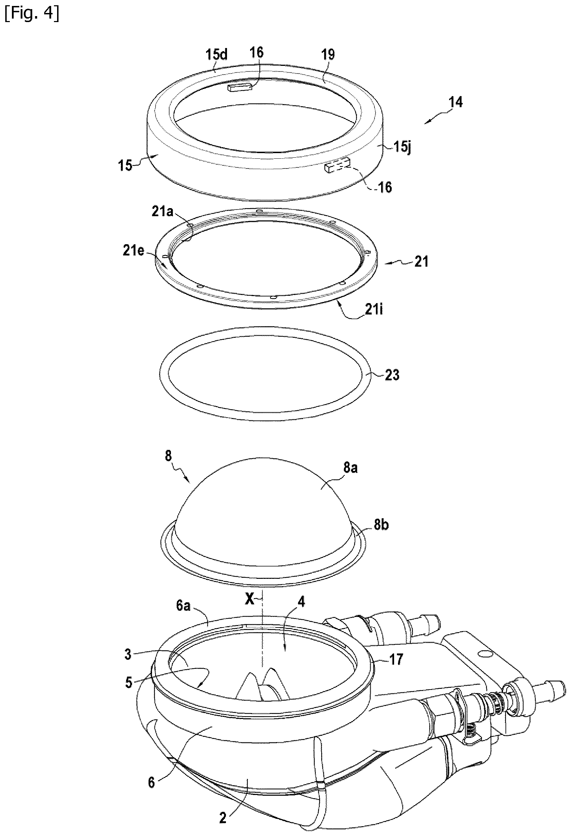

Le système d'assemblage par filetage **16, 17** de la bague de fixation **15** sur le corps **2** peut être réalisé de toute manière appropriée. Un tel système d'assemblage par filetage comporte au moins une nervure hélicoïdale ou filet **17** coopérant avec au moins une nervure hélicoïdale complémentaire ou filet complémentaire **16.** Le filet **17** est aménagé sur la bague de fixation **15** ou sur le corps **2** tandis que le filet complémentaire **16** est aménagé respectivement sur le corps **2** ou sur la bague de fixation **15.** Il est à noter que le filet **17** ou le filet complémentaire **16** peut être réalisé sous la forme d'un élément continu **(****Fig. 2****)** ou sous la forme d'éléments discontinus tels que des ergots ou des tétons **(****Fig. 4****).** De même, le système d'assemblage par filetage **16, 17** peut comporter plusieurs filets hélicoïdaux **17** et plusieurs filets hélicoïdaux complémentaires **16.**

Dans l'exemple de réalisation illustré sur la **Fig. 3****,** le corps **2** et en particulier la paroi tubulaire **6** présente extérieurement, au moins un filet **17** tandis que la bague de fixation **15** est pourvue d'un filet complémentaire **16** tel qu'un taraudage. Dans l'exemple illustré à la **Fig. 4****,** la bague de fixation **15** est équipée, en tant que filet complémentaire **16,** de deux ergots **16** venant coopérer avec le filet **17** pour assurer le vissage de la bague sur le corps. Il est à noter qu'il peut être prévu de réaliser sur la surface externe du corps **2** et en particulier sur la paroi tubulaire **6,** en tant que filet **17,** les ergots avec lesquels viennent coopérer au moins un filet complémentaire **16** de la bague de fixation **15.**

En position montée de la bague de fixation **15** sur le corps **2,** la partie périphérique **8b** de la membrane souple **8** se trouve interposée entre la bague de fixation **15** et le corps **2** et plus précisément la paroi tubulaire **6.** Le vissage de la bague de fixation **15** sur le corps **2** assure le maintien de la membrane souple **8** en appui étanche sur le corps **2.** Par exemple, la membrane souple **8** est dimensionnée de manière que sa partie périphérique **8b** se trouve positionnée en dehors du filet **17.**

Selon une caractéristique avantageuse de réalisation, la bague de fixation **15** est pourvue d'une rondelle **21** montée libre en rotation par rapport à la bague de fixation **15.** Cette rondelle **21** présente dans sa partie centrale, un trou **21a** de passage pour la partie centrale **8a** de la membrane souple. La section de ce trou de passage **21a** correspond sensiblement à la section du passage central **19** de la paroi annulaire de dessus **15d** ou comme dans l'exemple illustré est supérieure à cette section du passage central **19** de la paroi annulaire de dessus **15d.** En position montée, le bord interne de la rondelle **21** s'étend ainsi en retrait du bord interne de la paroi annulaire de dessus **15d** de la bague de fixation **15.**

Cette rondelle **21** qui comporte une face annulaire externe **21e** et une face annulaire interne **21i** est maintenue dans la bague de fixation **15** à l'intérieur d'un logement annulaire **22** délimité par la face interne de la paroi annulaire de dessus **15d** et par une nervure **15n** s'étendant en saillie à partir de la face interne **15i** de la jupe **15j.** Le logement **22** présente ainsi une section en forme de C. La rondelle **21** présente un déplacement transversal limité d'un côté par la face interne de la paroi annulaire de dessus **15d** sur laquelle peut venir en contact la face annulaire externe **21e** et de l'autre côté, par la nervure **15n** sur laquelle peut venir en contact la face annulaire interne **21i.** La rondelle **21** présente un déplacement radial limité par la face interne **15i** de la jupe **15j.** Il est à noter que la nervure **15n** peut être réalisée par le taraudage **16.** Ainsi, la rondelle **21** et la bague de fixation **15** sont liées entre elles par une liaison pivot selon l'axe passant par l'axe de symétrie de la rondelle.

Selon une caractéristique avantageuse de réalisation, la rondelle **21** est réalisée dans un matériau présentant un très grand pouvoir antiadhésif comme le polytétrafluoroéthylène (PTFE ou TEFLON) par exemple.

L'assemblage entre la rondelle **21** et la bague de fixation **15** peut être réalisé de toute manière appropriée. Une solution consiste à réaliser par une technique connue d'impression en trois dimensions, la bague de fixation **15** intégrant la rondelle **21.** La rondelle **21** peut également être mise en place par déformation au sein de la bague de fixation **15.**

Il doit être compris qu'en position non montée de la bague de fixation **15** sur le corps **2,** il existe une liberté de rotation entre la bague de fixation **15** et la rondelle **21.** En d'autres termes, la bague de fixation **15** peut tourner sur elle-même sans entraîner en rotation la rondelle **21** qui reste fixe.

Il est à noter qu'avant la mise en place de la bague de fixation **15,** la membrane souple **8** est positionnée pour recouvrir par sa partie périphérique **8b** la paroi annulaire de dessus **6a.** Lorsque la bague de fixation **15** est montée sur le corps **2,** la rondelle **21** n'est pas encore en contact avec la partie périphérique **8b** de la membrane souple **8** qui elle-même se trouve seulement en contact avec le rebord annulaire **6a** de la paroi tubulaire **6.** Lors de la rotation, la bague de fixation **15** et la rondelle **21** tournent simultanément. Lorsque la rondelle **21** se trouve positionnée de manière à venir en contact avec la partie périphérique **8b,** la membrane souple **8** se trouve alors interposée entre cette la rondelle **21** et le rebord annulaire **6a** de la paroi tubulaire **6. La** bague de fixation **15** continue alors son mouvement de rotation alors que la rondelle **21** reste fixe par rapport au rebord annulaire **6a** de la paroi tubulaire **6** et par rapport à la membrane souple **8.**

Lors de l'opération d'assemblage de la bague de fixation **15** sur le corps **2,** la bague de fixation **15** est entraînée en rotation sans entraîner en rotation la rondelle **21** qui maintient ainsi en place la membrane souple **8** sans la plisser. En fin de course de vissage de la bague de fixation **15,** cette dernière exerce par sa paroi annulaire de dessus **15d,** sur la face annulaire externe **21e,** un effort d'appui venant bloquer en position la rondelle **21** qui plaque la membrane souple **8** sur le rebord annulaire **6a** de la paroi tubulaire **6.**

La rondelle **21** est montée dans la bague de fixation **15** avec un débattement latéral limité pour exercer un effort de pression sur la membrane en position vissée de la bague de fixation **15** sur le corps **2.** En d'autres termes, la rondelle **21** est amenée en position finale de pression lors du vissage de la bague de fixation **15** par la mise en appui de la face interne de la paroi annulaire de dessus **15d** sur la face annulaire externe **21e** de la rondelle. La course de vissage de la bague de fixation **15** dépend bien entendu notamment du pas du filet **17** et du jeu séparant la face interne de la paroi annulaire de dessus **15d** et la face annulaire externe **21e** de la rondelle. Le déplacement latéral associé au déplacement radial décrit précédemment, permet d'aménager un espace dans lequel les liquides de nettoyage et de décontamination peuvent pénétrer et assurer leurs fonctions lors de phases de décontamination et de stérilisation.

Il est à noter que la rondelle **21** peut être en contact directement avec la membrane souple **8** ou comme dans l'exemple illustré, par l'intermédiaire d'un joint d'étanchéité plan ou annulaire **23** venant en appui sur la membrane souple **8** qui est en appui sur le rebord annulaire **6a.** Selon un exemple préféré de réalisation, le joint d'étanchéité **23** est monté sur la rondelle **21.** Tel que cela ressort de la **Fig. 3****,** la rondelle **21** comporte sur sa face annulaire interne **21i,** une gorge **21g** de montage pour le joint d'étanchéité **23.**

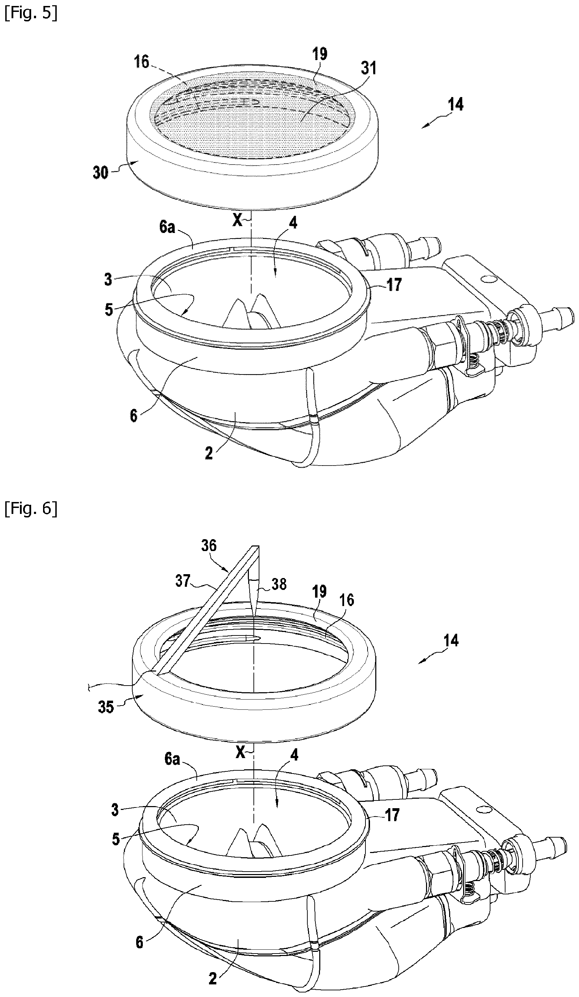

Lorsque la source **1** n'est pas utilisée pour produire des ondes ultrasonores, la bague de fixation **15** peut être retirée. En l'absence de la bague de fixation **15,** le corps **2** peut recevoir un couvercle de protection **30** pour protéger le transducteur ultrasonore **3.** Comme illustré à la **Fig. 5****,** le corps **2** comporte le filet **17** ou le filet complémentaire **16** coopérant en l'absence de la bague de fixation **15,** avec respectivement un filet complémentaire **16** ou un filet **17** du couvercle de protection **30.** Ce couvercle de protection **30** qui est de conception similaire à la bague de fixation **15** comporte en plus, une paroi de fermeture **31** venant fermer le passage central **19** de la bague de fixation **15.** Ce couvercle de protection **30** vient ainsi se visser sur la paroi annulaire **6** du corps **2** permettant de protéger le transducteur ultrasonore **6,** en dehors de ses périodes d'utilisation.

En l'absence de la bague de fixation **15** et du couvercle de protection **30,** le corps **2** est à même de recevoir un système d'alignement utilisable notamment lors d'une étape de calibration. Lors d'une étape de calibration, est utilisée une bague **35** analogue à la bague de fixation **15,** et équipée d'un système d'alignement 36. Par exemple, le système d'alignement **36** comporte un bras **37** porté par la bague et équipée à son extrémité d'un capteur **38** permettant de mesurer la pression acoustique générée par le transducteur ou de visualiser l'axe **X** de propagation des ondes acoustiques ou la zone de focalisation du transducteur ultrasonore **4.** Le corps **2** comporte le filet **17** ou le filet complémentaire **16** coopérant en l'absence de la bague de fixation **15** et du couvercle de protection **30,** avec respectivement un filet complémentaire **16** ou un filet **17** de la bague **35** équipée du système d'alignement **36.** La liaison mécanique ainsi obtenue entre le corps **2** et par suite le transducteur ultrasonore **3,** permet d'aligner sur l'axe acoustique **X** ou la zone de focalisation de la face d'émission **4** le capteur **38** mesurant la pression sur l'axe acoustique **X** ou indiquant sa position ou celle de la zone de focalisation.

Il ressort de la description qui précède que la source de production **1** et en particulier, la paroi tubulaire **6** du corps de la source, est adaptée pour assurer le montage par vissage, en fonction de l'utilisation de la source, soit de la bague de fixation **15** de la membrane souple **8,** soit d'un couvercle de protection **30** soit d'une bague **35** équipée d'un système d'alignement **36.** Les opérations de montage et de démontage sont particulièrement simples à mener à bien, sans risque de détérioration du transducteur ultrasonore **3.**

L'objet de l'invention propose ainsi un ensemble de production d'ondes ultrasonores comportant une source de production **1** conforme à l'invention et en tant qu'accessoires, une bague de fixation **15** d'une membrane souple, un couvercle de protection **30** et / ou une bague **35** équipée d'un système d'alignement **36,** chacun des accessoires comportant un filet complémentaire **16** coopérant avec le filet **17** de la paroi tubulaire **6** du corps de la source de production.

## Revendications

1. - Source de production d'ondes ultrasonores, comportant dans un corps **(2),** un transducteur ultrasonore **(3)** présentant, à l'avant, une face d'émission **(4),** le corps **(2)** comportant une ouverture **(5)** délimitée par une paroi tubulaire **(6),** et aménagée afin que la face d'émission **(4)** du transducteur ultrasonore **(3)** soit tournée vers l'ouverture **(5),** la paroi tubulaire **(6)** étant pourvue extérieurement d'au moins un filet **(17)** d'un système d'assemblage par filetage et destiné à coopérer avec au moins un filet complémentaire **(16)** soit d'une bague de fixation **(15)** d'une membrane souple **(8)** sur le corps possédant un passage central **(19)** pour une partie de la membrane souple **(8),** soit d'un couvercle de protection **(30),** soit d'une bague **(35)** équipée d'un système d'alignement **(36)** pour assurer le montage par vissage sur la paroi tubulaire **(6)** soit du couvercle de protection **(30),** soit de la bague **(35)** équipée du système d'alignement **(36),** soit de la bague de fixation **(15)** assurant par son vissage sur le corps **(2),** le maintien de la membrane souple **(8)** en appui étanche sur le corps **(2).**

2. - Source de production d'ondes ultrasonores selon la revendication 1, selon laquelle la paroi tubulaire **(6)** est pourvue à son extrémité, d'un rebord annulaire **(6a)** d'appui pour la membrane souple **(8).**

3. - Source de production d'ondes ultrasonores selon l'une des revendications 1 à 2, selon laquelle la bague de fixation **(15)** est pourvue d'une rondelle **(21)** montée libre en rotation relativement par rapport à la bague de fixation **(15)** afin de ne pas être entraîner en rotation lors de l'opération de vissage de la bague de fixation **(15),** cette rondelle exerçant un effort de pression sur la membrane souple **(8)** en position vissée de la bague de fixation **(15)** sur le corps **(2).**

4. - Source de production d'ondes ultrasonores selon la revendication 3, selon laquelle la rondelle **(21)** est montée dans la bague de fixation **(15)** avec un débattement latéral limité pour exercer un effort de pression sur la membrane souple **(8)** en position vissée de la bague de fixation **(15)** sur le corps **(2).**

5. - Source de production d'ondes ultrasonores selon la revendication 3 ou 4, selon laquelle la rondelle **(21)** est maintenue dans la bague de fixation **(15)** à l'intérieur d'un logement annulaire **(22)** de section en forme de C.

6. - Source de production d'ondes ultrasonores selon l'une des revendications 3 à 5, selon laquelle la rondelle **(21)** est équipée d'un joint d'étanchéité en contact avec la membrane souple **(8).**

7. - Source de production d'ondes ultrasonores selon l'une des revendications 3 à 6, selon laquelle la rondelle **(21)** est réalisée en polytétrafluoroéthylène.

8. - Source de production d'ondes ultrasonores selon l'une des revendications précédentes, selon laquelle le filet **(17)** ou le filet complémentaire **(16)** peut être réalisé sous la forme d'un élément continu ou sous la forme d'éléments discontinus tels que des ergots.

9. - Source de production d'ondes ultrasonores selon la revendication précédente, selon laquelle le filet **(17)** du corps **(2)** coopère avec un filet complémentaire **(16)** d'un couvercle de protection **(30).**

10. - Source de production d'ondes ultrasonores selon la revendication 1, selon laquelle le filet **(17)** du corps **(2)** coopère avec le filet complémentaire **(16)** d'une bague **(35)** équipée d'un système d'alignement **(36)** utilisable notamment lors d'une étape de calibration.

11. - Ensemble de production d'ondes ultrasonores comportant une source de production d'ondes ultrasonores selon l'une des revendications 1 à 10, comportant un corps possédant une paroi tubulaire **(6)** pourvue extérieurement d'au moins un filet **(17)** d'un système d'assemblage par filetage, cet ensemble comportant également, en tant qu'accessoires, une bague de fixation **(15)** d'une membrane souple, un couvercle de protection **(30)** et une bague **(35)** équipée d'un système d'alignement **(36),** chacun des accessoires comportant un filet complémentaire **(16)** coopérant avec le filet **(17)** de la paroi tubulaire **(6)** du corps de la source de production.

12. - Appareil de traitement par ondes ultrasonores, **caractérisé en ce qu'**il comporte une source (**1**) de production d'ondes ultrasonores selon l'une des revendications 1 à 10.

## Patentansprüche

1. Quelle zur Erzeugung von Ultraschallwellen, umfassend, in einem Körper (2), einen Ultraschall-Transducer (3), der auf der Vorderseite eine Emissionsseite (4) aufweist, wobei der Körper (2) eine Öffnung (5) umfasst, die von einer rohrförmigen Wand (6) begrenzt ist und so angeordnet ist, dass die Emissionsseite (4) des Ultraschall-Transducers (3) hin zur Öffnung (5) gedreht ist, wobei die rohrförmige Wand (6) an der Außenseite mit mindestens einem Gewinde (17) eines Systems zum Zusammenbau durch Gewinde versehen ist, und ausgelegt, um mit mindestens einem komplementären Gewinde (16) entweder eines Befestigungsrings (15) einer flexiblen Membran (8) auf dem Körper, der einen zentralen Durchgang (19) für einen Teil der flexible Membran (8) aufweist, oder eines Schutzdeckels (30) oder eines Rings (35) aufweist, der mit einem Ausfluchtungssystem (36) ausgestattet ist, um die Montage durch Verschrauben auf der rohrförmigen Wand (6) entweder des Schutzdeckels (30) oder des Rings (35), der mit dem Ausfluchtungssystem (36) ausgestattet ist, oder des Befestigungsrings (15) sicherzustellen, der durch seine Verschraubung auf dem Körper (2) die Beibehaltung der flexiblen Membran (8) in enger Auflage auf dem Körper (2) sicherstellt.

2. Quelle zur Erzeugung von Ultraschallwellen nach Anspruch 1, wobei die rohrförmige Wand (6) an ihrem Ende mit einem ringförmigen Rand (6a) zur Auflage für die flexible Membran (8) versehen ist.

3. Quelle zur Erzeugung von Ultraschallwellen nach einem der Ansprüche 1 bis 2, wobei der Befestigungsring (15) mit einer Unterlegscheibe (21) versehen ist, die in freier Drehung mit Bezug auf den Befestigungsring (15) montiert ist, um sich beim Vorgang des Verschraubens des Befestigungsrings (15) nicht in Drehung zu versetzen, wobei diese Unterlegscheibe eine Druckkraft auf die flexible Membran (8) in der verschraubten Position des Befestigungsrings (15) auf dem Körper (2) ausübt.

4. Quelle zur Erzeugung von Ultraschallwellen nach Anspruch 3, wobei die Unterlegscheibe (21) im Befestigungsring (15) mit einem seitlich begrenzten Federweg montiert ist, um eine Druckkraft auf die flexible Membran (8) in der verschraubten Position des Befestigungsrings (15) auf dem Körper (2) auszuüben.

5. Quelle zur Erzeugung von Ultraschallwellen nach Anspruch 3 oder 4, wobei die Unterlegscheibe (21) im Befestigungsring (15) im Inneren einer ringförmigen Aufnahme (22) mit einem C-förmigem Schnitt gehalten wird.

6. Quelle zur Erzeugung von Ultraschallwellen nach einem der Ansprüche 3 bis 5, wobei die Unterlegscheibe (21) mit einer Dichtung in Kontakt mit der flexiblen Membran (8) ausgestattet ist.

7. Quelle zur Erzeugung von Ultraschallwellen nach einem der Ansprüche 3 bis 6, wobei die Unterlegscheibe (21) aus Polytetrafluorethylen hergestellt ist.

8. Quelle zur Erzeugung von Ultraschallwellen nach einem der vorhergehenden Ansprüche, wobei das Gewinde (17) oder das komplementäre Gewinde (16) in Form eines kontinuierlichen Elements oder in Form von diskontinuierlichen Elementen wie z. B. Nocken hergestellt werden kann.

9. Quelle zur Erzeugung von Ultraschallwellen nach einem der vorhergehenden Ansprüche, wobei das Gewinde (17) des Körpers (2) mit einem komplementären Gewinde (16) eines Schutzdeckels (30) zusammenarbeitet.

10. Quelle zur Erzeugung von Ultraschallwellen nach Anspruch 1, wobei das Gewinde (17) des Körpers (2) mit dem komplementären Gewinde (16) eines Rings (35) zusammenarbeitet, das mit einem Ausfluchtungssystem (36) ausgestattet ist, das insbesondere bei einem Schritt des Kalibrierens verwendet werden kann.

11. Einheit zur Erzeugung von Ultraschallwellen, umfassend eine Quelle zur Erzeugung von Ultraschallwellen nach einem der Ansprüche 1 bis 10, umfassend einen Körper, der eine rohrförmige Wand (6) aufweist, die an der Außenseite mit mindestens einem Gewinde (17) eines Systems zum Zusammenbau durch Gewinde versehen ist, wobei diese Einheit auch, als Zubehör, einen Befestigungsring (15) einer flexiblen Membran, einen Schutzdeckel (30) und einen Ring (35) umfasst, der mit einem Ausfluchtungssystem (36) ausgestattet ist, wobei jedes der Zubehörteile ein komplementäres Gewinde (16) umfasst, das mit dem Gewinde (17) der rohrförmigen Wand (6) des Körpers der Quelle zur Erzeugung zusammenarbeitet.

12. Vorrichtung zur Behandlung durch Ultraschallwellen, **dadurch gekennzeichnet, dass** sie eine (1) Quelle zur Erzeugung von Ultraschallwellen nach einem der Ansprüche 1 bis 10 umfasst.

## Claims

1. An ultrasound production source comprising a body (2) containing an ultrasound transducer (3) presenting in front an emission face (4), the body (2) including an opening (5) defined by a tubular wall (6) and arranged so that the emission face (4) of the ultrasound transducer (3) faces towards the opening (5), the tubular wall (6) being provided externally with at least one thread (17) of a screw thread assembly system for co-operating with at least one complementary thread (16) either of a fastener ring (15) for fastening a flexible membrane (8) on the body, or of a protective cover (30), or of a ring (35) fitted with an alignment system (36), wherein the fastener ring (15) possesses a central passage (19) for passing a portion of the flexible membrane (8), the fastener ring (15), when screwed onto the body, serving to hold the flexible membrane (8) pressed in sealed manner against the body (2).

2. An ultrasound production source according to claim 1, wherein the tubular wall (6) is provided at its end with an annular rim (6a) against which the flexible membrane (8) bears.

3. An ultrasound production source according to claim 1 to 2, wherein the fastener ring (15) is provided with a washer (21) mounted to turn freely relative to the fastener ring (15) so as to avoid being turned while screwing on the fastener ring (15), the washer exerting a pressure force on the flexible membrane (8) when the fastener ring (15) is screwed in position on the body (2).

4. An ultrasound production source according to claim 3, wherein the washer (21) is mounted in the fastener ring (15) with limited freedom to move laterally in order to exert a pressure force on the flexible membrane (8) when the fastener ring (15) is screwed in position on the body (2).

5. An ultrasound production source according to claim 3 or claim 4, wherein the washer (21) is held in the fastener ring (15) inside an annular recess (22) of C-shaped section.

6. An ultrasound production source according to any one of claims 3 to 5, wherein the washer (21) is fitted with a sealing gasket in contact with the flexible membrane (8).

7. An ultrasound production source according to any one of claims 3 to 6, wherein the washer (21) is made of polytetrafluoroethylene.

8. An ultrasound production source according to any preceding claim, wherein the thread (17) or the complementary thread (16) may be made in the form of a continuous element or in the form of discontinuous elements such as studs.

9. An ultrasound production source according to preceding claim 1, wherein the thread (17) of the body (2) co-operates with a complementary thread (16) of a protective cover (30).

10. An ultrasound production source according to claim 1, wherein the thread (17) of the body (2) co-operates with the complementary thread (16) of a ring (35) fitted with an alignment system (36) suitable for use in particular during a calibration step.

11. An ultrasound production assembly comprising an ultrasound production source according to any one of claims 1 to 10 comprising a body having a tubular wall (6) provided externally with at least one thread (17) of a screw thread assembly system, the assembly also comprising, as accessories: a fastener ring (15) for fastening a flexible membrane; a protective cover (30); and a ring (35) fitted with an alignment system (36); each of the accessories including a complementary thread (16) co-operating with the thread (17) of the tubular wall (6) of the body of the production source.

12. Ultrasound treatment apparatus, **characterized in that** it includes an ultrasound production source (1) according to any one of claims 1 to 10.
